# EUROPEAN PATENT SPECIFICATION

(11) **EP 0 666 920 B1**
(45) Date of publication and mention of the grant of the patent: **16.09.1998**
(21) Application number: 93923634.5
(22) Date of filing: 28.10.1993
(51) Int. Cl.: C12N 15/57, C12N 9/68, C12N 5/10, C12N 1/19, A61K 38/48

(54) **THROMBIN ACTIVATABLE PLASMINOGEN DERIVATIVES**
PLASMINOGEN-DERIVATE DIE DURCH THROMBIN AKTIVIERBAR SIND
DERIVES DE PLASMINOGENE CAPABLES D'ETRE ACTIVES PAR LA THROMBINE

(30) Priority: 29.10.1992 GB 9222758
(43) Date of publication of application: 16.08.1995
(73) Proprietor: BRITISH BIOTECH PHARMACEUTICALS LIMITED, Cowley Oxford, OX4 5LY (GB)
(72) Inventor: GILBERT, Richard James,, Cowley, Oxford OX4 5LY (GB); HUNTER, Michael George, Cowley, Oxford OX4 5LY (GB); DAWSON, Keith Martyn, Cowley, Oxford OX4 5LY (GB)
(74) Representative: Walls, Alan James
(86) International application number: GB9302219
(87) International publication number: WO9410318

(56) References cited:
- WO-A-91/09118
- JOURNAL OF BIOLOGICAL CHEMISTRY, vol. 266, no. 12, 25 April 1991, BALTIMORE US, pages 7353-7358; K. NAITO AND K. FUJIKAWA: 'Activation of human blood coagulation factor XI independent of factor XII'
- SCIENCE, vol. 253, August 1991, LANCASTER, PA US, pages 909-912; D. GAILANI AND G. BROZE: 'Factor XI activation in a revised model of blood coagulation'
- BIOCHEMISTRY, vol. 25, 1986, EASTON, PA US, pages 2417-2424; K. FUJIKAWA ET AL: 'Amino acid sequence of human factor XI, a blood coagulation factor with four tandem repeats that are highly homologous with plasma prekallikrein'
- CHEMICAL ABSTRACTS, vol. 114, no. 13, 1 April 1991, Columbus, Ohio, US; abstract no. 119257; D. ABERCROMBIE ET AL: 'Fibrin specific thrombolysis by two-chain urokinase-type plasminogen activator cleaved after arginine 156 by thrombin' page 502; & TROMB. HAEMOSTASIS, vol. 64, no. 3, 1990, pages 426-432
- JOURNAL OF BIOLOGICAL CHEMISTRY, vol. 261, no. 8, 15 March 1986, BALTIMORE US,pages 3486-3489; A. ICHINOSE ET AL: 'The activation of pro-urokinase by plasma kallikrein and its inactivation by thrombin'
- AGRICULTURAL AND BIOLOGICAL CHEMISTRY, vol. 52, no. 2, February 1988, TOKYO JP, pages 329-336; Y. HIBINO ET AL: 'Enhanced expression of human pro-urokinase cDNA in E. coli'
- NATURE, vol. 339, 29 June 1989, pages 721-723; E. MADISON ET AL: 'Serpin-resistant mutants of human tissue-type plasminogen activator'

## Description

This invention is an improvement of the invention disclosed in our copending patent application WO-A-9109118, and relates to plasminogen analogues which are activated by thrombin to have fibrinolytic activity or to inhibit blood clot formation. It also relates to nucleic acid (DNA and RNA) coding for all or part of such compounds. The invention also relates to their preparation, pharmaceutical compositions containing them and their use in the treatment of thrombotic disease.

Plasminogen is a key component of the fibrinolytic system which is the natural counterpart to the clotting system in the blood. In the process of blood coagulation, a cascade of enzyme activities is involved in generating a fibrin network which forms the framework of a clot, or thrombus. Degradation of the fibrin network (fibrinolysis) is accomplished by the action of the enzyme plasmin. Plasminogen is the inactive precursor of plasmin and conversion of plasminogen to plasmin is accomplished by cleavage of the peptide bond between arginine 561 and valine 562 of plasminogen. Under physiological conditions this cleavage is catalysed by tissue-type plasminogen activator (tPA) or by urokinase-type plasminogen activator (uPA).

If the balance between the clotting and fibrinolytic systems becomes locally disturbed, intravascular clots may form at inappropriate locations leading to conditions such as coronary thrombosis and myocardial infarction, deep vein thrombosis, stroke, peripheral arterial occlusion and embolism. In such cases, the administration of fibrinolytic agents has been shown to be a beneficial therapy for the promotion of clot dissolution. Antithrombotic agents are also useful for the prevention of clot formation.

However, the problem with the majority of agents used for fibrinolytic treatment is that at clinically useful doses they are not thrombus specific, as they activate plasminogen in the general circulation. An alternative approach to enhancing fibrinolysis is disclosed in our copending patent application WO-A-9109118, and is based on the use of molecules activatable to have fibrinolytic activity or to inhibit clot formation. The activation is catalysed by one or more endogenous enzymes involved in blood clotting. An advantage of this approach is that thrombus selectivity of fibrinolysis or inhibition of clot formation activity is achieved by way of the thrombus-specific localisation of the activating enzyme. In particular, WO-A-9109118 discloses, *inter alia,* plasminogen analogues activatable to plasmin by cleavage by thrombin.

Thrombin (E.C. 3.4.21.5) is a serine protease which catalyses the proteolysis of a number of proteins including fibrinogen (A alpha and B beta chains), Factor XIII, Factor V, Factor VII, Factor VIII, protein C and antithrombin III. The structure required for recognition by thrombin appears to be partially determined by the local amino acid sequence around the cleavage site, but is also determined to a variable extent by sequence(s) remote from the cleavage site. For example, in the fibrinogen A alpha chain, residues P2 (Val), P9 (Phe) and P10 (Asp) are crucial for α-thrombin-catalysed cleavage at the Arg(16)-Gly(17) peptide bond (Ni, F. et al 1989, Biochemistry 28 3082-3094). WO-A-9109118 discloses that optimum cleavage sites for alpha-thrombin may have the structure (i) P4-P3-Pro-Arg-P1'-P2'
where each of P3 and P4 is independently a residue of a hydrophobic amino acid (such as valine) and each of P1' and P2' is independently a non-acidic amino acid residue, or (ii) P2-Arg-P1'
where P2 or P1' is a glycine residue. Accordingly, the thrombin activatable plasminogen analogue compounds disclosed as preferred in WO-A-9109118, and all those specifically exemplified therein, have cleavage sites conforming to the foregoing structures (i) or (ii). The data reported in WO-A-910918 suggests that, of the specifically exemplified thrombin-cleavable plasminogen analogues, that designated T19 is the most effective in the assay systems used. It has a cleavage site based on Factor XIII, Pro (559) and Gly (560) of wild-type plasminogen having been replaced by Val-Glu-Leu-Gln-Gly-Val-Val-Pro. The thrombin cleavage site of T19, the most effective of the compounds specifically exemplified, therefore conforms with the requirements of structure (i) above - preferred according to WO-A-9109118.

In the absence of cofactors, Factor XI has been reported not to be cleaved by thrombin (Naito, K. and Fujikawa, K (1991), J. Biol. Chem. 266 7353-7358), or to be only slowly cleaved with a k_{cat}/Kₘ=1.6x10⁵M-1min-1 (Gailani, D. and Broze, G.J. 1991, Science **253**, 909-912). The cleavage site sequence of this thrombin substrate differs from the preferred general formulae i) and ii) of WO-A-9109118. In factor XI, a basic amino acid, Lys, is in the P3 position. However, although thrombin cleavage activity at this site in factor XI is very low compared to that of Factor XIII (k_{cat}/Kₘ=1.4x10⁵M-1sec-1; Naski et al., 1991, Biochemistry **30** 934-941), it has now been found in accordance with this invention, that plasminogen analogues having a thrombin cleavage sequence with a basic amino acid residue in the P3 position have surprisingly increased activity compared to T19. Such novel analogues are cleaved more rapidly by thrombin, and exhibit increased activity in a linked chromogenic assay. More significantly, such analogues are active in a plasma clot lysis assay, which more closely resembles conditions *in vivo.*

A further example of a thrombin cleavage site where P3 is a basic amino acid residue is found in single-chain urokinase, where P3 is arginine. Cleavage at this site produces inactive two-chain urokinase (Ichinose et al., (1986) J. Biol. Chem. **261** 3486-9). In the absence of cofactors, cleavage of single-chain urokinase by thrombin is also moderately slow, with a k_{cat}/Kₘ=3.8x10⁴M-1sec-1; (de Munk et al., 1990 Fibrinolysis **4** 161); however, plasminogen analogues bearing the urokinase cleavage site have now been shown to have increased activity compared to T19.

Accordingly, the present invention is an improvement of the invention disclosed in WO-A-9109118 in that it provides a plasminogen analogue which is activatable by thrombin to have plasmin activity (as generally disclosed in WO-A-9109118) but specifically characterised in that it comprises a thrombin-cleavable site sequence P4-P3-Pro-Arg-P1'-P2' wherein P3 is a residue of a basic amino acid, P4 is a residue of a hydrophobic amino acid, and each of P1' and P2' is independently a non-acidic amino acid residue, said site being cleavable by thrombin between Arg and P1'.

Plasminogen has been numbered according to the protein sequencing studies of Sottrup-Jensen et al. (in: Atlas of Protein Sequence and Structure (Dayhoff, M.O., ed.) 5 suppl. 3, p.95 (1978)) which indicated that plasminogen was a 790 amino acid protein and that the site of cleavage was the Arg(560)-Val(561) peptide bond. However, a suitable plasminogen cDNA useful in this embodiment of the invention and that isolated by Forsgren et al (FEBS Letters 213 254-260 (1987)) code for a 791 residue protein with an extra Ile at position 65. In this specification, the numbering of the amino acids in plasminogen corresponds to that of the cDNA used. There may be polymorphism in the structure of plasminogen and there may be forms of plasminogen in which the numbering of the cleavage site differs but it is intended that such variants be included in the embodiment.

Therefore the term "plasminogen analogue", as used in this specification, means a molecule differing from wild type plasminogen and having the ability to be cleaved or otherwise acted on to form a molecule having plasmin activity.

Plasminogen analogues within the scope of this embodiment of the invention retain the fibrin binding activity of wild type plasminogen to an adequate degree but may have altered inhibition characteristics; preferred plasminogen analogues have a plasma half life which is comparable with that of wild type plasminogen, but this property is not essential.

In the thrombin-cleavable site sequence present in plasminogen analogues according to the invention, the basic amino acid residue P3 may be a lysine or arginine residue: the hydrophobic amino acid residue P4 may be a valine, isoleucine or leucine residue; and each of the non acidic amino acid residues P1' and P2' may independently be a valine or isoleucine residue.

In one embodiment of the invention, the basic amino acid residue P3 is a lysine residue and the hydrophobic amino acid residue P4 is isoleucine. In another embodiment, in addition to P3 being lysine and P4 being isoleucine, the non-acidic residues P1' and P2' are isoleucine and valine respectively.

In another embodiment of the invention, the basic amino acid residue P3 is an arginine residue and the hydrophobic amino acid residue P4 is leucine. In yet another embodiment, in addition to P3 being arginine and P4 being leucine, the non-acidic residues P1' and P2' are both valine.

Particular plasminogen analogues within the scope of the invention contain the cleavage site Ile-Lys-Pro-Arg-P1'-P2', Thr-Thr-Lys-Ile-Lys-Pro-Arg-P1'-P2', or the cleavage site Leu-Arg-Pro-Arg-P1'-P2', where each of P1' and P2' is independently a non-acidic amino acid residue. As mentioned, P1' and P2' may be isoleucine or valine residues. Other particular plasminogen analogues within the scope of the invention contain the cleavage site Val-Glu-Leu-Gln-Gly-Leu-Arg-Pro-Arg-Val-Val, Gly-Gln-Lys-Thr-Leu-Arg-Pro-Arg-Val-Val, Ala-Gly-Gln-Lys-Thr-Leu-Arg-Pro-Arg-Val-Val, Ala-Leu-Arg-Pro-Arg-Val-Val or Ala-Thr-Thr-Lys-Ile-Lys-Pro-Arg-Ile-Val.

Specific and preferred compounds according to the invention are plasminogen analogues in which relative to wild-type plasminogen:
a) Pro(559)-Gly(560) are replaced by Thr-Thr-Lys-Ile-Lys-Pro and Val(562) is replaced by Ile. In this analogue, amino acid 563 is valine as in the wild type. This mutant has been designated BB10151.
b) Cys(558)-Pro(559)-Gly(560) are replaced by Ala-Gly-Gln-Lys-Thr-Leu-Arg-Pro; and Cys(566) is replaced with Ala. In this analogue, amino acids 562 and 563 are valine as in the wild type. This mutant has been designated BB10156.
c) Cys(558)-Pro(559)-Gly(560) are replaced by Ala-Leu-Arg-Pro; and Cys(566) is replaced with Ala. In this analogue. amino acids 562 and 563 are valine as in the wild type. This mutant has been designated BB10170.
d) Pro(559)-Gly(560) are replaced by Val-Glu-Leu-Gln-Gly-Leu-Arg-Pro. In this analogue, amino acids 562 and 563 are valine as in the wild type. This mutant has been designated BB10171
e) Cys(558)-Pro(559)-Gly(560) are replaced by Ala-Thr-Thr-Lys-Ile-Lys-Pro; Val(562) is replaced by Ile; and Cys(566) is replaced with Ala. This mutant has been designated BB10158
f) Pro(559)-Gly(560) are replaced by Gly-Gln-Lys-Thr-Leu-Arg-Pro.

Plasminogen analogues in accordance with the invention have been defined by particular reference to the nature of their thrombin-cleavable site sequence, since it is the surprising rapidity of cleavage at that site which underlies the improved thrombolytic activity of the compounds. However, it is likely that plasminogen analogues in accordance with the invention (i.e. containing the now identified novel cleavage site sequences) may contain other modifications (as compared with wild type plasminogen) which may be one or more additions, deletions or substitutions at sites more or less remote from the cleavage site, without losing the benefit of rapid cleavage. These addition, deletion or substitution mutations are thus outside of the domain of the compound corresponding to the serine protease domain of wild-type plasminogen. An example of such a modification would be the addition, removal, substitution or alteration of one or more kringle domains to alter fibrin binding activity or reduce α2-antiplasmin binding. A specific example would be mutation of the lysine-binding site located on Kringle 1 to interfere with the binding of α2-antiplasmin to this site. Such variants may be resistant to inhibition by α2-antiplasmin. Preferred embodiments include BB10189, BB10190 and BB10192, which are the plasminogen analogues BB10153, BB010170 and BB10171 respectively with the additional mutations of Asp 137> Ser and Asp 139> Ser.

Another example would be mutation to prevent disulphide bond formation between Cys (558) and Cys (566), for example by deleting or replacing one or both cysteine residues in order to remove the constraint put on the cleavage site by the disulphide bond formed between the cysteine residues. In one embodiment of the invention one or both of the cysteines is replaced with alanine or serine residues. Of the preferred embodiments described above, variants BB10156, BB10158 and BB10170 have such open-loop modifications.

An example of a modification involving deletion would be lys-plasminogen variants of a plasminogen analogue in which the amino terminal 68, 77 or 78 amino acids have been deleted. Such variants may have enhanced fibrin binding activity as has been observed for lys-plasminogen compared to wild-type glu-plasminogen (Bok, R. A. and Mangel, W. F. 1985, Biochemistry 24 3279-3286). A further example involving deletion would be variants of a plasminogen analogue in which the kringle 1 or kringle 1-4 domains have been deleted to impair α2-antiplasmin binding. Such variants may be resistant to inhibition by α2-antiplasmin. Deletion of kringles 1-4 would also alter the fibrin binding and pharmokinetic properties of the molecule.

For the highly clot selective analogue of plasminogen of the present invention it may be preferred to introduce a mutation in the serine protease domain that interferes with plasmin inhibitor binding (SEQ ID No. 2 depicts the serine protease domain of wild-type plasmin, and references herein to that domain, where numbered, use the numbering of SEQ ID No. 2). This mutation could be in a position analogous to that shown to prevent inhibitor binding to tissue plasminogen activator (Madison, E. L. et al 1989 Nature 339 721-724). Particular plasminogen analogues with additional mutations within the serine protease domain of plasmin are Glu(606) to Lys or Glu(623) to Lys. In one embodiment of the invention, addition, deletion or substitution mutations are in residues in a region corresponding to residues 22 or 24 of the protease domain of plasmin. Particular plasminogen analogues with additional mutations at positions 22 or 24 of the serine protease domain are those that have one or both of the following mutations: Phe(583) to Arg or Met(585) to Arg. Alternatively it could be in another position which prevents inhibitor binding to plasminogen. Such additions, deletions or substitutions can be in one or more residues in close spatial proximity, other than sequence proximity, to a site of interaction between the compound and antiplasmin. Suitable plasminogen analogues would have said sequence additions, deletions or substitutions in a residue or residues in a region corresponding to residues 17-20, 44-54, 62, 154, 158, or 198-213 of the protease domain of plasmin (using the numbering of SEQ ID No 2); such modifications are described in co-pending patent application WO-A-9403614, which discloses endopeptidases of the chymotrypsin superfamily which exhibit resistance to serine protease inhibitors. Such resistance is provided by a modification in the endopeptidase or its precursor which induces one of the following
a) a conformational change in the local fold of the protease;
b) a change in the relative orientations of the protease and inhibitor on forming a complex;
c) a change in the steric bulk of the protease in the region of the inhibitor,
d) a change in the electrostatic potential field in the region of the inhibitor binding site; or
e) any combination of the above.

Suitable plasminogen analogues of the invention posses one or more of the following mutations: Glu-62 to Lys or Ala, Ser-17 to Leu, Arg 19 to Glu or Ala, or Glu-45 to Lys, Arg or Ala, of the serine protease domain of wild -type plasminogen (SEQ ID No. 2).

A preferred embodiment is BB 10158 with the A4 mutation (Glu606 to Lys) described in WO-A-9403614 (BB10199). This mutation is designed to interrupt ionic interactions on the surface of plasminogen, interfering with binding to antiplasmin. Mutagenesis was carried out using a 24 base oligonucleotide 5'CTT GGG GAC TTC TTC AAG CAG TGG3', designed to convert Glu606 to Lys. Other preferred embodiments have, either singly or in combination, mutations at Glu606, Glu623, Phe583, Met585 or Lys 607. The Glu606 and Glu623 mutations were exemplified in WO-A-9403614. An example of this embodiment is BB10153 which is the plasminogen analogue BR10151 with the additional mutations of Glu606 to Lys and Glu623 to Lys.

Other plurally-modified plasminogen analogues in accordance with the invention may include one or more modifications to prevent, reduce or alter glycosylation patterns. Plasminogen analogues incorporating such modifications may have a longer half-life, reduced plasma clearance and/or higher specific activity.

Preferred features of plasminogen analogues within the scope of the invention also apply, where appropriate, to other compounds of the invention, *mutatis mutandis*.

The plasminogen analogues of the first aspect of the invention can be synthesised by any convenient route. According to a second aspect of the invention there is provided a process for the preparation of such a plasminogen analogue, the process comprising coupling successive amino acid residues together and/or ligating oligopeptides. Although proteins may in principle be synthesised wholly or partly by chemical means, it is preferred to prepare them by ribosomal translation, preferably in vivo, of a corresponding nucleic acid sequence. The protein may be glycosylated appropriately.

It is preferred to produce proteins of the invention by using recombinant DNA technology. DNA encoding a naturally occurring plasminogen may be obtained from a cDNA or genomic clone or may be synthesised. Amino acid substitutions, additions or deletions are preferably introduced by site-specific mutagenesis. DNA sequences encoding plasminogen analogues may be obtained by procedures familiar to those skilled in the art of genetic engineering.

The process for producing proteins using recombinant DNA technology will usually include the steps of inserting a suitable coding sequence into an expression vector and transferring the vector into a host cell. Therefore, according to a third aspect of the invention, there is provided synthetic or recombinant nucleic acid coding for a proteinaceous compound as described above. The nucleic acid may be RNA or DNA and may be in the form of a vector, such as a plasmid, cosmid or phage. The vector may be adapted to transfect or transform prokaryotic (for example bacterial) cells and/or eukaryotic (for example yeast or mammalian) cells. A vector will comprise a cloning site and usually at least one marker gene. An expression vector will have a promoter operatively linked to the sequence to be inserted in the cloning site, and, preferably, a sequence enabling the protein product to be secreted.

The plasminogen analogues of the invention may be expressed using a vector of the type described in WO-A-9109118, which comprises a first nucleic acid sequence coding for a protein or embodying a cloning site, operatively linked to a second nucleic acid sequence containing a strong promoter and enhancer sequence derived from human cytomegalovirus, a third nucleic acid sequence encoding a polyadenylation sequence derived from SV40 and a fourth nucleic acid sequence coding for a selectable marker expressed from an SV40 promoter and having an additional SV40 polyadenylation signal at the 3' end of the selectable marker sequence.

It is to be understood that the term "vector" is used in this specification in a functional sense and is not to be construed as necessarily being limited to a single nucleic acid molecule. So, for example, the first, second and third sequences of the vector defined above may be embodied in a first nucleic acid molecule and the fourth sequence may be embodied in a second nucleic acid molecule.

This vector enables the plasminogen analogues to be expressed and secreted into the cell culture medium in a biologically active form without the need for any additional biological or chemical procedures.

According to a third aspect of the invention, there is provided a process for the preparation of nucleic acid encoding the plasminogen analogues described above, the process comprising coupling successive nucleotides together and/or ligating oligo- and/or poly-nucleotides.

In a further aspect of the invention, there is provided a cell or cell line transformed by nucleic acid and/or a vector as described above. Suitable cells or cell lines to be transformed include both prokaryotic (for example *Escherichia coli*) and eukaryotic cells, such as yeast cells (including *Saccharomyces cerevisiae* and *Pichia pastoris*) and mammalian cells. Mammalian cells which grow in continuous culture and which can be transfected or otherwise transformed by standard techniques are preferred. Examples of suitable cells include Chinese hamster ovary (CHO) cells, mouse myeloma cell lines such as NS0 and P3X63-Ag8.653, COS cells, HeLa cells, BHK cells, melanoma cell lines such as the Bowes cell line, mouse L cells, human hepatoma cell lines such as Hep G2, mouse fibroblasts and mouse NIH 3T3 cells. CHO cells are particularly preferred as hosts for the expression of plasminogen and plasminogen analogues.

Transformation may be achieved by any convenient method; electroporation is particularly suitable.

Plasminogen analogues of the present invention may be used for the prophylaxis and/or treatment of conditions caused by an imbalance between clotting and fibrinolysis, the method comprising administering to a patient an effective amount of the plasminogen analogue. Therefore, according to a further aspect of the invention, there is provided a plasminogen analogue as disclosed herein, for use in medicine, particularly in the prophylaxis and/or treatment of conditions caused by an imbalance between clotting and fibrinolysis, where it is desired to produce local fibrinolytic and/or anticoagulant activity. Such conditions include myocardial and cerebral infarction, arterial and venous thrombosis, thromboembolism, post-surgical adhesions, thrombophlebitis and diabetic vasculopathies and coagulation imbalances associated with cancer.

The invention also provides the use of a plasminogen analogue as disclosed herein in the preparation of a thrombolytic, antithrombotic or thrombolytic antithrombotic agent.

Furthermore, there is also provided a pharmaceutical or veterinary composition comprising one or more plasminogen analogues as disclosed herein and a pharmaceutically or veterinarily acceptable carrier. Such a composition may be adapted for administration orally, by intravenous or intramuscular injection or by infusion. Suitable injectable compositions include preparations of sterile plasminogen analogue(s) in isotonic physiological saline and/or buffer and may also include a local anaesthetic to alleviate the pain of injection. Similar compositors may be used for infusion. Where the compound is to be administered as a topical treatment, it may be formulated as a cream, ointment or lotion in a suitable base.

The compounds of the invention may be supplied in unit dosage form, for example as a dry powder or water-free concentrate in a hermetically sealed container such as an ampoule or sachet.

The quantity of material to be administered will depend on the amount of fibrinolysis or inhibition of clotting required, the required speed of action, the seriousness of the thromboembolic position and the size of the clot. The precise dose to be administered will, because of the very nature of the condition which compounds of the invention are intended to treat, be determined by the physician. As a guideline, however, a patient being treated for a mature thrombus will generally receive a daily dose of a plasminogen analogue of from 0.01 to 10 mg/kg of body weight either by injection in for example up to 5 doses or by infusion.

The following figures and examples of the invention are offered by way of illustration, and not by way of limitation. In the drawings referred to in the examples:
Figure 1 shows the rate of cleavage of BB10151 (T51) by thrombin;
Figure 2 shows the results of a chromogenic assay comparing the activation of BB10151 (T51) and T19 by thrombin;
Figure 3 shows the clot lysis activity of BB10151 (T51).

### Example 1 - Construction Expression and Purification of BB10151

The isolation of plasminogen cDNA and construction of the vectors pGWH and pGWHgP have been described in WO-A-9109118. In pGWHgP, transcription through the plasminogen cDNA can initiate at the HCMV promoter/enhancer and the selectable marker gpt is employed.

The techniques of genetic manipulation, expression and protein purification used in the manufacture of the modified plasminogen example to follow, are well known to those skilled in the art of genetic engineering. A description of most of the techniques can be found in one of the following laboratory manuals: "Molecular Cloning" by T. Maniatis, E.F. Fritsch and J. Sambrook published by Cold Spring Harbor Laboratory, Box 100, New York, or "Basic Methods in Molecular Biology" by L.G. Davis, M.D. Dibner and J.F. Battey published by Elsevier Science publishing Co Inc, New York.

### Additional and modified methodologies are detailed in the methods section below.

BB10151 is a plasminogen analogue in which the amino acid residues Pro(559)-Gly(560) of wild-type plasminogen are replaced by Thr-Thr-Lys-Ile-Lys-Pro and Val(562) is replaced by Ile to produce a cleavage loop cleavable by thrombin (SEQ ID NO. 1). This site is based on a potential thrombin cleavage site in factor XI. The procedures used in this example are essentially as described in WO-A-9109118 Examples 2 and 3, with the mutagenesis reaction carried out on the 1.87kb KpnI to HincII fragment of plasminogen cloned into the bacteriophage M13mp18. Single stranded template was prepared and the mutation made by oligonucleotide directed mutagenesis. In this case a 48 base long oligonucleotide
(5'CACCCCCCTACGATTCTAGGTTTAATTTTAGTTGTACATTTCTTCGGC3') (SEQ ID No. 4) was used to direct the mutagenesis.

Plasmid DNA was introduced into CHO cells by electroporation using 800 V and 25 µF. Selective medium containing 250 µl/ml xanthine, 5 µg/ml mycophenolic acid, 1x hypoxanthine-thymidine (HT)) was added to the cells 24 hours post transfection and the medium was changed every two to three days. Plates yielding gpt-resistant colonies were screened for plasminogen production using an ELISA assay. Cells producing the highest levels of antigen were re-cloned and the best producers scaled up into flasks with production being carefully monitored. Frozen stocks of all these cell lines were laid down. Producer cells were scaled up into roller bottles to provide conditioned medium from which plasminogen protein was purified using lysine SEPHAROSE 4B. (The word SEPHAROSE is a trade mark.) The cell line used to produce this mutant protein was 123.C6.

### Example 2 - Construction of BB10153

BB10153 is a derivative of BB10151 containing two additional mutations (Glu606 to Lys and Glu623 to Lys) to impair binding of α2-antiplasmin. The 663bp EcoRV to Sph I fragment of BB10151 (cloned in pUC - see Example 1) was removed and replaced with the equivalent 663bp fragment from the antiplasmin resistant mutant A3A4. Construction of this is described in example 5 of WO-A-9403614. The 24 base oligonucleotide 5'CTT GGG GAC TTC TTC AAG CAG TGG3' (SEQ ID No. 3), was used to convert Glu-606 to Lys and the 27 base oligonucleotide 5'GTTCGAGATTCACTTTTTGGTGTGCAC3' (SEQ ID No. 5) was used to convert Glu623 to Lys. The full length plasminogen was then cloned into the expression vector pGW1H prior to the insertion of the gpt selection marker as described in WO-A-9109118 Example 2.

### Example 3 - Construction of BB10156, BB10158 & BB10170

The DNA encoding plasminogen mutant BB10150 was used as the template for production of BB10156, BB10158 & BB10170. BB10150 is a plasminogen analogue in which the amino acid residues Pro(559)-Gly(560) of wild-type plasminogen are replaced by Val-Val-Pro and has the additional mutations Cys(558) to Ala and Cys(566) to Ala to prevent disulphide bond formation. The opened cleavage loop sequence of BB10150 is shown in SEQ ID No. 6. BB10150 was made by mutagenesis using two oligonucleotide primers (5' CTAGGTACAACCGCTTTCTTCGGCT 3'(SEQ ID No. 7) and 5' GGTGGGCCACCGCCCCCCCCAC 3' (SEQ ID No. 8) and the 1.87kb KpnI to HincII fragment of mutant T13 (see patent application WO-A-9109118, Example 13 and SEQ ID No. 9) cloned into M13.

Plasminogen analogues BB10156, BB10158 and BB10170 were all constructed by site specific mutagenesis using the previously described mutant BB10150 in M13 as the template so that they all have the same Cys to Ala mutations at residues 558 and 566. BB10156 is a plasminogen analogue in which the amino acid residues Pro(559)-Gly(560) are replaced by Gly-Gln-Lys-Thr-Leu-Arg-Pro (SEQ ID No.10). BB 10158 is a plasminogen analogue in which the amino acid residues Pro(559)-Gly(560) are replaced by Thr-Thr-Lys-Ile-Lys-Pro and Val(562) is replaced by Ile (SEQ ID No. 11). BB10170 is a plasminogen analogue in which the amino acid residues Pro(559)-Gly(560) are replaced by Leu-Arg-Pro (SEQ ID No. 12). The oligonucleotides used to prime each mutagenesis are shown below:-

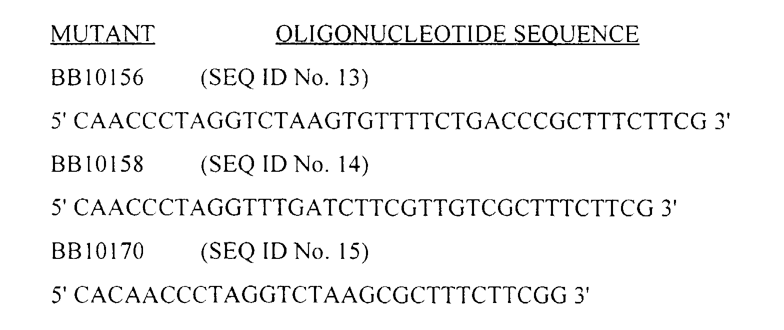

In each case, following DNA sequencing, the mutation was cloned directly into the pGW1Hg.plasminogen expression vector using the restriction enzymes HindIII and SplI. These sites had previously been introduced at the extreme 5' end of plasminogen and at 1850 respectively via mutagenesis; the plasminogen amino acid coding sequence was not affected by this procedure.

### Example 4 - Construction of BB10169 and BB10171

BB10169 is a plasminogen analogue in which the amino acid residues Pro(559)-Gly(560) of wild-type plasminogen are replaced by Val-Glu-Leu-Gln-Gly-Ile-Lys-Pro and Val(562) is replaced by Ile (SEQ ID No. 16). BB10169 was made by oligonucleotide directed mutagenesis of BB10151 in M13 using the 42 base oligonucleotide
5' GATTCTAGGTTTAATGCCCTGCAGTTCCACACATTTCTTCGG 3' (SEQ ID No. 17) followed by cloning into pGW1Hg plasminogen using Hindlll and SplI. BB10171 is a plasminogen analogue in which the amino acid residues Pro(559)-Gly(560) are replaced by Val-Glu-Leu-Gln-Gly-Leu-Arg-Pro (SEQ ID No. 18). The single stranded M13 from BB10169 was used as the template for the construction of BB10171. Mutagenesis was primed using the 39 base oligonucleotide
5' CACCCCCCTACCACTCTGGGTCTCAGGCCCTGCAGTTCC 3' (SEQID No. 19) and, following DNA sequencing, was cloned into the expression vector using Hindlll and SplI.

### Example 5 - Construction and Expression of BB10189, BB10190 and BB10191

Plasminogen analogues BB10189, BB10190 and BB10191 have the kringle 1 double mutation Asp(137) to Ser. Asp(139) to Ser to disable the lysine binding site. The mutation was performed in the BB10153, BB10170 and BB10171 backgrounds (see examples 2, 3 & 4) using the 28 base long oligonucleotide
5' CCCTGCGGAGAGTTGGATGGATTCCTGC 3' (SEQ ID No. 20).

The mutation was then cloned into pGW1Hg.plasminogen using the restriction enzymes HindIII and SplI.

### Example 6 - Construction of BB10181

BB10181 has the cleavage site sequence of BB10170 and an additional mutation of Phe(583) to Arg to interfere with the binding of α2-antiplasmin. BB10181 was constructed via an intermediate BB10150-based construct using M13 containing full length BB10150 as the template and the oligonucleotide
5' GAAGTGCATTCCTCTCCTCGTACGAAG 3' (SEQ ID No. 21) as the primer. The mutated BB10150 gene was then cloned into pGW1Hg using the restriction enzymes HindIII and Smal. The BB10181 expression vector was then made from this intermediate by replacing the HindIII to SplI fragment from this plasmid with the corresponding portion from BB10170 (see example 3).

### Example 7 - Construction of BB10186

BB10186 has the cleavage site sequence of BB10171 and an additional mutation of Met(585) to Arg to interfere with the binding of α2-antiplasmin. BB10186 was made in a similar manner to that described in example 6 above for BB10181. The intermediate BB10150 construct was made using the 25 base long oligonucleotide
5' CCACAGAAGTGTCTTCCAAACCTCG 3' (SEQ ID No. 22) followed by cloning into pGW1Hg. BB10186 was then made by a fragment switch using the Hindlll to SplI fragment from BB10171 (see example 4).

### Example 8 - Construction of BB10199

BB10199 has the cleavage site sequence of BB10158 and an additional mutation of Glu(606) to Lys to interfere with the binding of α2-antiplasmin. BB10199 was made essentially as described in WO-A-9403614 examples 2 & 3. The KpnI to EcoRV fragment of BB10158 (see example 3 above) was used to replace the corresponding fragment ofa BB10151 Glu(606) to Lys construct cloned into pUC and was then cloned into the final expression vector as described in WO-A-9403614.

### Example 9 - Cleavage of BB10151

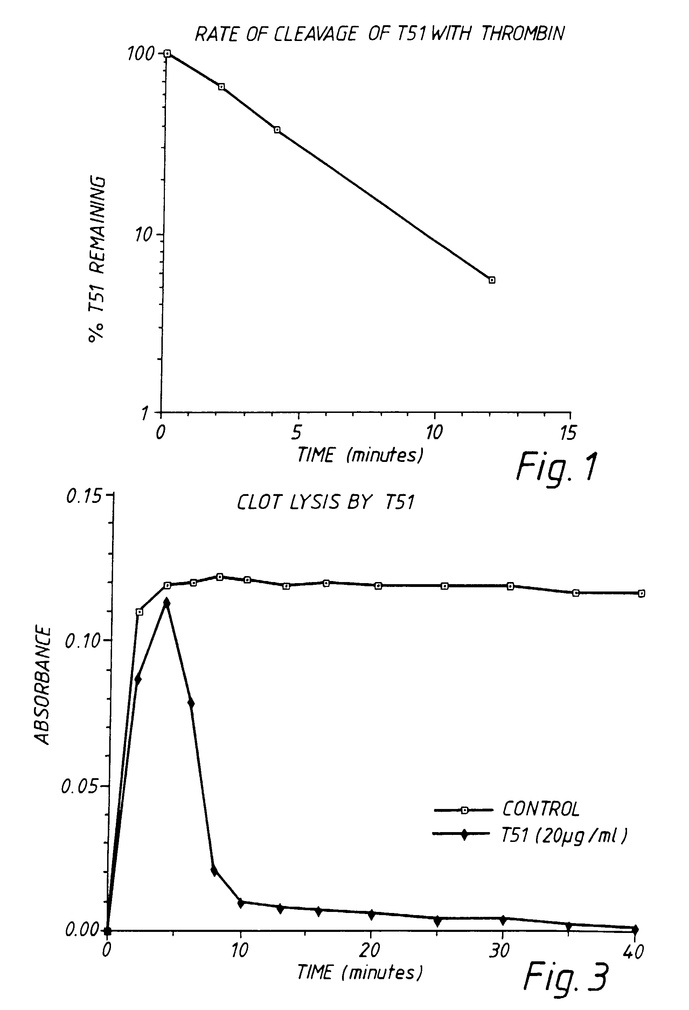

Plasminogen mutants (12.5µg) were incubated with 2.8µg thrombin as described in Method 1. The time course of cleavage of the plasminogen mutants was determined by quantitative gel scanning; 50% cleavage times for T19 and BB10151 were 9 and 3 minutes respectively. Gel scan data for cleavage of BB10151 are shown in Figure 1.

### Example 10 - Activation of BB10151

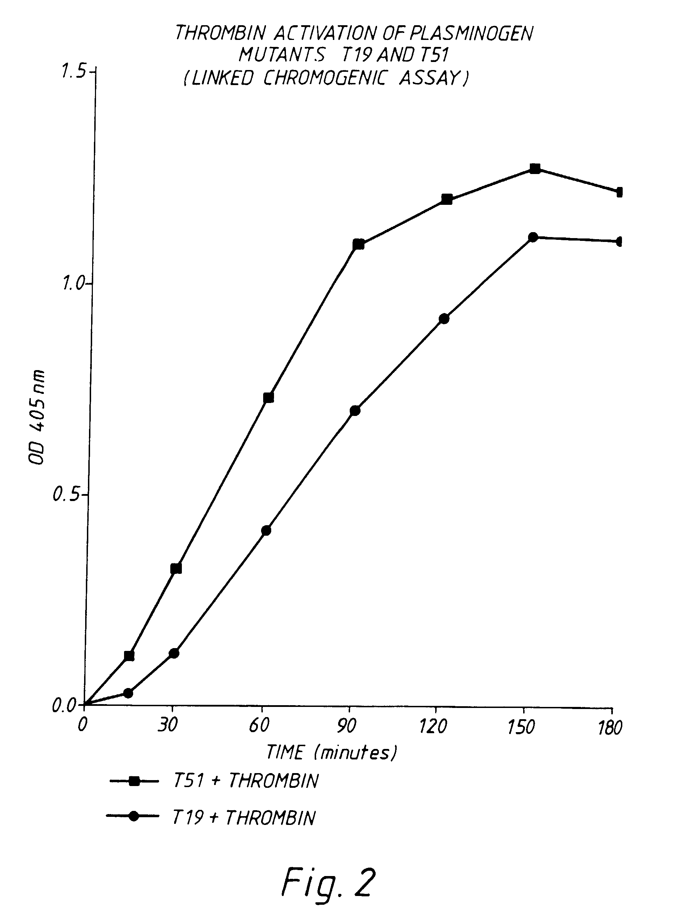

Purified BB10151 protein was assayed for activation using the linked chromogenic assay (see Method 2.1). Results of this assay are shown in Figure 2 in which the increase in absorbance at 405nm with time demonstrates that plasmin activity is generated upon incubation of BB10151 with thrombin. T19 is shown for comparison and was found to be approximately 2 times less potent than BB10151 in this assay.

### Example 11 - Plasma Clot Lysis

The ability of BB10151 and T19 to lyse a plasma clot was determined as described in Method 2.2 and the results of such an assay are shown in Figure 3. BB10151 (20µg/ml) was able to cause complete lysis of the clot whereas T19 did not lyse the clot at concentrations up to 150µg/ml. Thus BB10151 was found to be at least 7 times more active than T19 at inducing lysis of a plasma clot.

Representative examples of other plasminogen analogues of the invention were compared for plasma clot lysis activity at a concentration of 40µg/ml and the results in Table 1 show that they possess similar activity to BB10151.

**Table 1**

| Plasminogen Analogue | Time for 50% clot lysis (min) |
|---|---|
| BB10151 | 4.5 |
| T19 | not lysed |
| BB10158 | 12.5 |
| BB10199 | 6 |
| BB10153 | 4.1 |
| BB10171 | 4.4 |
| BB10156 | 6.6 |
| BB10170 | 3.1 |

### Methods

### 1. Cleavage Analysis

Plasminogen analogues are assessed for susceptibility to cleavage by thrombin using SDS PAGE under reducing conditions. Typical incubation volumes of 0.125 ml in 100mM Tris HCl pH 7.4 consist of plasminogen analogue, at the concentration shown in the examples. and thrombin, at the concentration shown in the examples. Incubations are performed at 37°C. Control incubations are performed under the same conditions in the absence of thrombin. The activation reactions were stopped by precipitating the protein by the addition of trichloroacetic acid to a final concentration of 20% and standing at 4°C for >4 hours. The precipitates were then pelleted, washed with acetone and resuspended in SDS PAGE sample buffer (0.1m Tris pH6.8. 10% glycerol, 1% SDS, 0.5% mercaptoethanol and 0.05% bromophenol blue). The samples were analysed either on 8-25% gradient gels or 12% gels. The resulting gels were analysed using a SHIMADZU Gel Scanner which scans the gel and calculates the concentration of protein in bands by determining the area under the peaks. (The word SHIMADZU is a trade mark.) The rate of cleavage of plasminogen was thus determined by measuring the disappearance of the plasminogen band at approximately 92kDa and the appearance of the plasmin heavy chain band at approximately 66kDa.

### 2. Activation Analysis

### 2.1 Linked Chromogenic Assay

Plasminogen analogue and thrombin are incubated together in the presence of the chromogenic substrate S2251 and plasmin produced by activation directly cleaves the S2251 leading to an increase in absorbance at 405nm. The assay is performed in a total volume of 880µl in a buffer containing 50mM Tris HCI, 0.1mM EDTA, 0.005% TRITON X100 and 0.1% HSA (the word TRITON is a trademark). S2251 is added to a final concentration of 0.35mg/ml and the plasminogen analogue concentration used is 3 µg/ml. The thrombin concentration used is 4.55 NIHU/ml. Aliquots of 100µl of the reaction are removed during incubation at 37°C and added to 25µl 4% acetic acid, in microtitre plates, to stop the reaction. At the completion of the time course the plates are read on a microplate reader at a wavelength of 405nm.

### 2.2 In Vitro Plasma Clot Lysis Assay

A mixture of 50µl rabbit plasma (anticoagulated with 3.8% trisodium citrate), 50µl APTT reagent (Instrumentation Labs) and an appropriate volume of plasminogen analogue in 0.1M Tris HCl pH 7.4 is made up to 200µl with the same buffer in a well of a 96 well microtitre plate. A separate well contains 4.4µl 500mM CaCl₂ mixed with 50.6µl of the same buffer. The plate is incubated at 37°C for 30 minutes and clotting is initiated by transferring 50µl of the CaCl₂ to the well containing plasminogen analogue. Progress of clot formation and dissolution is followed by measuring the absorbance at 405nm (620nm reference) at timed intervals during continued incubation at 37°C for 1 hour.

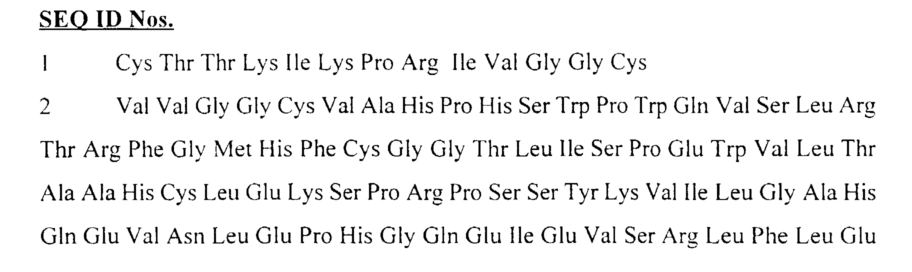

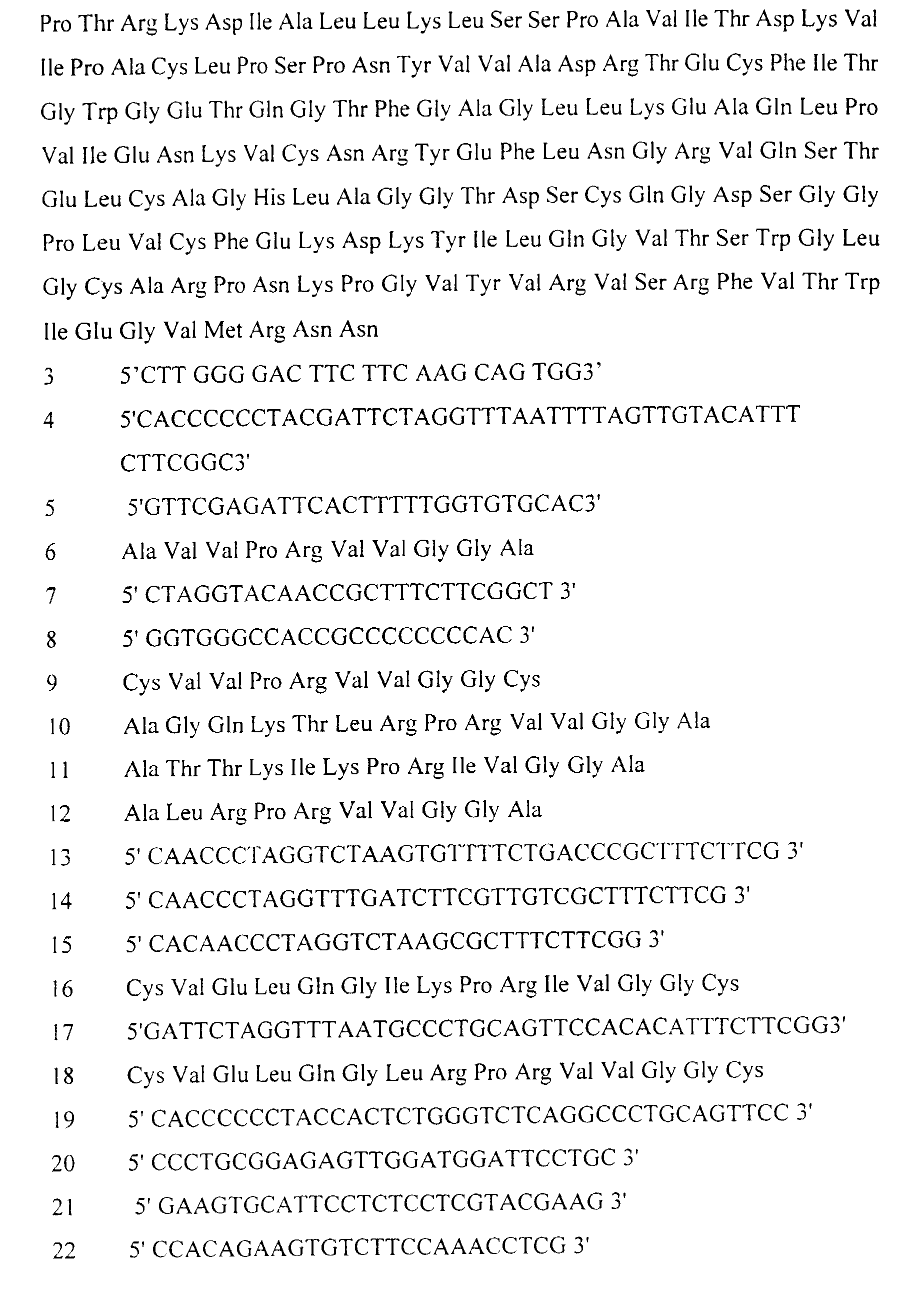

## Claims

1. A plasminogen analogue activatable by thrombin to have plasm in activity, which contains the cleavage site sequence
P4-P3-Pro-Arg-P1'-P2'
where P3 is a basic amino acid residue, P4 is a hydrophobic amino acid residue and each of P1' and P2' is independently a non-acidic amino acid residue, said site being cleavable by thrombin between Arg and P1'.

2. A compound as claimed in claim 1 wherein the basic amino acid residue P3 is a lysine or arginine residue.

3. A compound as claimed in claim 1 or claim 2 wherein the hydrophobic amino acid residue P4 is isoleucine or leucine.

4. A compound as claimed in any of claims 1-3 wherein the non-acidic amino acid residues P1' and P2' are each independently valine or isoleucine .

5. A compound as claimed in any of claims 1-4 wherein the basic amino acid residue P3 is a lysine residue and the hydrophobic amino acid residue P4 is isoleucine.

6. A compound as claimed in claim 5 wherein the non-acidic amino acid residues P1' and P2' are isoleucine and valine respectively.

7. A compound as claimed in claim I which contains the cleavage site Ile-Lys-Pro-Arg-P1'-P2' where each of P1' and P2' is independently a non-acidic amino acid.

8. A compound as claimed in claim 7 which contains the cleavage site sequence Thr-Thr-Lys-Ile-Lys-Pro-Arg-Ile-Val.

9. A compound as claimed in claim 8 wherein Pro(559)-Gly(560) of wild-type plasminogen is replaced by Thr-Thr-Lys-Ile-Lys-Pro and Val(562) is replaced by Ile.

10. A compound as claimed in claim 7 which contains the cleavage site sequence Ala-Thr-Thr-Lys-Ile-Lys-Pro-Arg-Ile-Val.

11. A compound as claimed in any of claims 1-4 wherein the basic amino acid residue P3 is an arginine residue and the hydrophobic amino acid residue P4 is leucine.

12. A compound as claimed in claim 11 wherein the non-acidic amino acid residues P1' and P2' are both valine.

13. A compound as claimed in claim 1 which contains the cleavage site sequence Leu-Arg-Pro-Arg-P1'-P2' where each of P1' and P2' is independently a non-acidic amino acid.

14. A compound as claimed in claim 13 which contains the cleavage site sequence Val-Glu-Leu-Gln-Gly-Leu-Arg-Pro-Arg-Val-Val.

15. A compound as claimed in claim 14 wherein Pro(559)-Gly(560) of wild-type plasminogen is replaced by Val-Glu-Leu-Gln-Gly-Leu-Arg-Pro.

16. A compound as claimed in claim 13 which contains the cleavage site sequence Gly-Gln-Lys-Thr-Leu-Arg-Pro-Arg-Val-Val.

17. A compound as claimed in claim 16 wherein Pro(559)-Gly(560) of wild-type plasminogen is replaced by Gly-Gln-Lys-Thr-Leu-Arg-Pro.

18. A compound as claimed in claim 13 which contains the cleavage site sequence Ala-Gly-Gln-Lys-Thr-Leu-Arg-Pro-Arg-Val-Val.

19. A compound as claimed in claim 13 which contains the cleavage site sequence Ala-Leu-Arg-Pro-Arg-Val-Val.

20. A compound as claimed in any one of claims 1-19 which, in addition to said cleavage site sequence, comprises additions, deletions or substitutions relative to wild-type plasminogen.

21. A compound as claimed in claim 20 in which one or both amino acids corresponding to Cys(558) and Cys(566) of wild-type plasminogen are replaced or deleted.

22. A compound as claimed in claim 21 wherein one or both of the saidresidues are replaced by an alanine or serine residue(s).

23. A compound as claimed in any one of claims 20-22 wherein the said sequence additions, deletions or substitutions are within the domain of the compound corresponding to the serine protease domain of wild-type plasminogen SEQ ID No. 2.

24. A compound as claimed in any one of claims 20-22 wherein the said sequence additions, deletions or substitutions are outside the domain of the compound corresponding to the serine protease domain of wild-type plasminogen SEQ ID No. 2.

25. A compound as claimed in claim 23 in which the said sequence additions, deletions or substitutions are in one or more residues in close spatial proximity, other than sequence proximity. to a site of interaction between the compound and antiplasmin.

26. A compound as claimed in claim 25, which has one or more of the following mutations: Glu-62 to Lys or Ala, Ser-17 to Leu, Arg 19 to Glu or Ala, or Glu-45 to Lys, Arg or Ala, of the serine protease domain of wild -type plasminogen (SEQ ID No. 2).

27. A compound as claimed in claim 23 in which the said sequence additions. deletions or substitutions are in a residue or residues in a region corresponding to residues 22 or 24 of the protease domain of plasmin (using the numbering of SEQ ID No. 2).

28. A compound as claimed in claim 27, which has one or both of the following mutations: Phe(583) to Arg or Met(585) to Arg.

29. A compound as claimed in claim 25 in which the amino acid residue corresponding to Glu(606) of wild-type plasminogen is substituted by a lysine residue.

30. A compound as claimed in claim 25 in which the amino acid residue corresponding to Glu(623) of wild-type plasminogen is substituted by a lysine residue.

31. A compound as claimed in claim 24 corresponding to lys-plasminogen in which the N-terminal domain has been deleted.

32. A compound as claimed in claim 24 in which the amino acid residues corresponding to the amino terminal 68, 77 or 78 amino acid residues of wild-type plasminogen have been deleted.

33. A compound as claimed in claim 24 in which one or more kringle domains corresponding to the kringle domains of wild-type plasminogen are deleted.

34. A compound as claimed in claim 24 containing one or both of the following mutations; Asp(137) to Ser or Asp(139) to Ser.

35. A compound as claimed in claim 1 wherein Pro(559)-Gly(560) of wild-type plasminogen is replaced by Thr-Thr-Lys-Ile-Lys-Pro; Val(562) is replaced by Ile; Glu(606) is replaced by Lys and Glu(623) is replaced by Lys.

36. A compound as claimed in claim 1 wherein Cys(558)-Pro(559)-Gly(560) of wild-type plasminogen is replaced by Ala-Gly-Gln-Lys-Thr-Leu-Arg-Pro and Cys(566) is replaced by Ala.

37. A compound as claimed in claim 1 wherein Cys(558)-Pro(559)-Gly(560) of wild-type plasminogen is replaced by Ala-Leu-Arg-Pro and Cys(566) is replaced by Ala.

38. A compound as claimed in claim 1 wherein Cys(558)-Pro(559)-Gly(560) of wild-type plasminogen is replaced by Ala-Thr-Thr-Lys-Ile-Lys-Pro; Val(562) is replaced by Ile; and Cys(566) is replaced by Ala.

39. A nucleic acid encoding a compound as claimed in any one of claims 1-38.

40. Nucleic acid as claimed in claim 39 which is a vector.

41. A vector as claimed in claim 40 which is a plasmid, cosmid or phage.

42. Nucleic acid as claimed in claim 40 or 41 comprising a first nucleic acid sequence coding for a protein or embodying a cloning site, operatively linked to a second nucleic acid sequence containing a strong promoter and enhancer sequence derived from human cytomegalovirus, a third nucleic acid sequence encoding a polyadenylation sequence derived from SV40 and a fourth nucleic acid sequence coding for a selectable marker expressed from an SV40 promoter and having an additional SV40 polyadenylation signal at the 3' end of the selectable marker sequence.

43. A cell or cell line transformed by nucleic acid as claimed in any of claims 39-42.

44. A cell as claimed in claim 43, which is a mammalian Chinese hamster ovary (CHO) cell.

45. A cell or cell line as claimed in claim 43, which is one selected from the group consisting of: a mouse myeloma NS0 cell line, a COS cell or a BHK cell.

46. A compound as claimed in any one of claims 1-38 for use in the prophylaxis and/or treatment of conditions caused by an imbalance between clotting and fibrinolysis.

47. The use of a compound as claimed in any one of claims 1-38 in the preparation of an agent for the prophylaxis and/or treatment of conditions caused by an imbalance between clotting and fibrinolysis.

48. A pharmaceurical or veterinary composition comprising one or more modified plasminogen analogue(s) as claimed in any one of claims 1-38 together with a pharmaceutically or veterinarily acceptable carrier.

## Patentansprüche

1. Durch Thrombin aktivierbares Plasminogen-Analog, das Plasmin-Aktivität besitzt, enthaltend die Spaltstellensequenz
P4-P3-Pro-Arg-P1'-P2',
worin P3 ein basischer Aminosäurerest ist, P4 ein hydrophober Aminosäurerest ist und P1' und P2' jeweils unabhängig voneinander ein nicht-saurer Aminosäurerest sind, wobei die Stelle zwischen Arg und P1' durch Thrombin spaltbar ist.

2. Verbindung nach Anspruch 1, worin der basische Aminosäurerest P3 ein Lysin- oder Arginrest ist.

3. Verbindung nach Anspruch 1 oder 2, worin der hydrophobe Aminosäurerest P4 Isoleucin oder Leucin ist.

4. Verbindung nach einem der Ansprüche 1 bis 3, worin die nicht-sauren Aminosäurereste P1' und P2' jeweils unabhängig voneinander Valin oder Isoleucin sind.

5. Verbindung nach einem der Ansprüche 1 bis 4, worin der basische Aminosäurerest P3 ein Leucinrest ist und der hydrophobe Aminosäurerest P4 Isoleucin ist.

6. Verbindung nach Anspruch 5, worin die nicht-sauren Aminosäurereste P1' und P2' jeweils Isoleucin und Valin sind.

7. Verbindung nach Anspruch 1, die die Spaltstelle Ile-Lys-Pro-Arg-P1'-P2' enthält, worin P1' und P2' jeweils unabhängig voneinander eine nicht-saure Aminosäure sind.

8. Verbindung nach Anspruch 7, die die Spaltstellensequenz Thr-Thr-Lys-Ile-Lys-Pro-Arg-Ile-Val enthält.

9. Verbindung nach Anspruch 8, worin Pro559-Gly560 von Wildtyp-Plasminogen durch Thr-Thr-Lys-Ile-Lys-Pro ausgetauscht sind und Val562 durch Ile ausgetauscht ist.

10. Verbindung nach Anspruch 7, die die Spaltstellensequenz Ala-Thr-Thr-Lys-Ile-Lys-Pro-Arg-Ile-Val enthält.

11. Verbindung nach einem der Ansprüche 1 bis 4, worin der basische Aminosäurerest P3 ein Argininrest ist und der hydrophobe Aminosäurerest P4 Leucin ist.

12. Verbindung nach Anspruch 11, worin die nicht-sauren Aminosäurereste P1' und P2' beide Valin sind.

13. Verbindung nach Anspruch 1, die die Spaltstellensequenz Leu-Arg-Pro-Arg-P1'-P2' enthält, worin P1' und P2' jeweils unabhängig voneinander eine nicht-saure Aminosäure sind.

14. Verbindung nach Anspruch 13, die die Spaltstellensequenz Val-Glu-Leu-Gln-Gly-Leu-Arg-Pro-Val-Val enthält.

15. Verbindung nach Anspruch 14, worin Pro559-Gly560 von Wildtyp-Plasminogen durch Val-Glu-Leu-Gln-Gly-Leu-Arg-Pro ausgetauscht sind.

16. Verbindung nach Anspruch 13, die die Spaltstellensequenz Gly-Gln-Lys-Thr-Leu-Arg-Pro-Arg-Val-Val enthält.

17. Verbindung nach Anspruch 16, worin Pro559-Gly560 von Wildtyp-Plasminogen durch Gly-Gln-Lys-Thr-Leu-Arg-Pro ausgetauscht sind.

18. Verbindung nach Anspruch 13, die die Spaltstellensequenz Ala-Gly-Gln-Lys-Thr-Leu-Arg-Pro-Arg-Val-Val enthält.

19. Verbindung nach Anspruch 13, die die Spaltstellensequenz Ala-Leu-Arg-Pro-Arg-Val-Val enthält.

20. Verbindung nach einem der Ansprüche 1 bis 19, die zusätzlich zu der Spaltstellensequenz Additionen, Deletionen oder Substitutionen relativ zum Wildtyp-Plasminogen umfaßt.

21. Verbindung nach Anspruch 20, bei der eine oder beide Aminosäuren, die Cys558 und Cys566 von Wildtyp-Plasminogen entsprechen, ausgetauscht oder deletiert sind.

22. Verbindung nach Anspruch 21, bei der einer oder beide Reste durch einen Alanin- oder Serinrest/reste ausgetauscht ist/sind.

23. Verbindung nach einem der Ansprüche 20 bis 22, bei der sich Sequenz-Additionen, -Deletionen oder - Substitutionen in der Domäne der Verbindung, die der Serinprotease-Domäne von Wildtyp-Plasminogen (SEQ. ID Nr. 2) entspricht, befinden.

24. Verbindung nach einem der Ansprüche 20 bis 22, bei der sich Sequenz-Additionen, -Deletionen oder - Substitutionen außerhalb der Domäne der Verbindung, die der Serinprotease-Domäne von Wildtyp-Plasminogen (SEQ. ID Nr. 2) entspricht, befinden.

25. Verbindung nach Anspruch 23, bei der sich Sequenz-Additionen, -Deletionen oder -Substitutionen in einem oder mehreren Resten befinden, die in enger räumlicher Nähe, außer der Sequenznähe, zu der Wechselwirkungsstelle zwischen der Verbindung und Antiplasmin sind.

26. Verbindung nach Anspruch 25, die eine oder mehrere der folgenden Mutationen hat: Glu62 zu Lys oder Ala, Ser17 zu Leu, Arg19 zu Glu oder Ala oder Glu45 zu Lys, Arg oder Ala der Serinprotease-Domäne von Wildtyp-Plasminogen (SEQ. ID Nr. 2).

27. Verbindung nach Anspruch 23, bei der sich Sequenz-Additionen, -Deletionen oder -Substitutionen in einem Rest oder in Resten befinden, die in einem Bereich liegen, der den Resten 22 oder 24 der Protease-Domäne von Plasmin entspricht (unter Verwendung der Numerierung von SEQ. ID Nr. 2).

28. Verbindung nach Anspruch 27, die eine oder mehrere der folgenden Mutationen hat: Phe583 zu Arg oder Met585 zu Arg.

29. Verbindung nach Anspruch 25, bei der der Aminosäurerest, der Glu606 von Wildtyp-Plasminogen entspricht, durch einen Lysinrest ausgetauscht ist.

30. Verbindung nach Anspruch 25, bei der der Aminosäurerest, der Glu623 von Wildtyp-Plasminogen entspricht, durch einen Lysinrest substituiert ist.

31. Verbindung nach Anspruch 24, die Lys-Plasminogen entspricht, bei der die N-terminale Domäne deletiert worden ist.

32. Verbindung nach Anspruch 24, bei der die Aminosäurereste, die den aminoterminalen Aminosäureresten 68, 77 oder 78 von Wildtyp-Plasminogen entsprechen, deletiert worden sind.

33. Verbindung nach Anspruch 24, bei der eine oder mehrere Kringle-Domänen, die den Kringle-Domänen von Wildtyp-Plasminogen entsprechen, deletiert sind.

34. Verbindung nach Anspruch 24, enthaltend ein oder mehrere der folgenden Mutationen: Asp137 zu Ser oder Asp139 zu Ser.

35. Verbindung nach Anspruch 1, worin Pro559-Gly560 von Wildtyp-Plasminogen durch Thr-Thr-Lys-Ile-Lys-Pro ausgetauscht sind, Val562 durch Ile, Glu606 durch Lys und Glu623 durch Lys ausgetauscht ist.

36. Verbindung nach Anspruch 1, worin Cys558-Pro559-Gly560 von Wildtyp-Plasminogen durch Ala-Gly-Gln-Lys-Thr-Leu-Arg-Pro ausgetauscht sind und Cys566 durch Ala ausgetauscht ist.

37. Verbindung nach Anspruch 1, worin Cys558-Pro559-Gly560 von Wildtyp-Plasminogen durch Ala-Leu-Arg-Pro ausgetauscht sind und Cys566 durch Ala ausgetauscht ist.

38. Verbindung nach Anspruch 1, worin Cys558-Pro559-Gly560 von Wildtyp-Plasminogen durch Ala-Thr-Thr-Lys-Ile-Lys-Pro ausgetauscht sind, Val562 durch Ile und Cys566 durch Ala ausgetauscht ist.

39. Nucleinsäure, die eine Verbindung nach einem der Ansprüche codiert.

40. Nucleinsäure nach Anspruch 39, die ein Vektor ist.

41. Vektor nach Anspruch 40, der ein Plasmid, Cosmid oder Phage ist.

42. Nucleinsäure nach Anspruch 40 oder 41, umfassend eine erste Nucleinsäuresequenz, die ein Protein codiert oder eine Klonierungsstelle umfaßt, die operativ an eine zweite Nucleinsäuresequenz gebunden ist, die einen starken Promoter und eine von menschlichem Cytomegalovirus abstammende Enhancersequenz enthält, eine dritte Nucleinsäuresequenz, die eine von SV40 abstammende Polyadenylierungssequenz codiert und eine vierte Nucleinsäuresequenz, die einen selektierbaren Marker codiert, der durch einen SV40 Promotor exprimiert wird und ein zusätzliches SV40 Polyadenylierungs-Signal am 3' Ende der selektierbaren Markersequenz hat.

43. Zelle oder Zellinie, die mit einer Nucleinsäuresequenz nach einem der Ansprüche 39 bis 42 transformiert ist.

44. Zelle nach Anspruch 43, die eine Säuger-Eierstockzelle des chinesischen Hamsters (CHO) ist.

45. Zelle oder Zellinie nach Anspruch 43, die ausgewählt ist aus der Gruppe, bestehend aus: einer Maus-Myelom NSO-Zellinie, einer COS-Zelle oder einer BHK-Zelle.

46. Verbindung nach einem der Ansprüche 1 bis 38 zur Verwendung bei der Prophylaxe und/oder Behandlung von Zuständen, die durch eine Gleichgewichtsstörung zwischen der Gerinnung und Fibrinolyse verursacht werden.

47. Verwendung einer Verbindung nach einem der Ansprüche 1 bis 38 bei der Herstellung eines Mittels zur Prophylaxe und/oder Behandlung von Zuständen, die durch eine Gleichgewichtsstörung zwischen Gerinnung und Fibrinolyse verursacht werden.

48. Pharmazeutische oder veterinäre Zusammensetzung, umfassend ein oder mehrere modifizierte Plasminogen-Analog/Analoga nach einem der Ansprüche 1 bis 38, zusammen mit einem pharmazeutisch oder veterinär annehmbaren Träger.

## Revendications

1. Analogue de plasminogène qui peut être activé par de la thrombine pour avoir une activité de plasmine, ledit analogue contenant la séquence de sites de clivage
P4-P3-Pro-Arg-P1'-P2'
dans laquelle P3 est un résidu d'acide aminé alcalin, P4 est un résidu d'acide aminé hydrophobe et chacun des sites P1' et P2' est indépendamment un résidu d'acide aminé non acide, ledit site pouvant être clivé par de la thrombine entre Arg et P1'.

2. Composé selon la revendication 1, dans lequel le résidu d'acide aminé alcalin P3 est un résidu de lysine ou d'arginine.

3. Composé selon la revendication 1 ou 2, dans lequel le résidu d'acide aminé hydrophobe P4 est l'isoleucine ou la leucine.

4. Composé selon l'une quelconque des revendications 1 à 3, dans lequel les résidus d'acides aminés non acides P1' et P2' sont chacun indépendamment de la valine ou de l'isoleucine.

5. Composé selon l'une quelconque des revendications 1 à 4, dans lequel le résidu d'acide aminé alcalin P3 est un résidu de lysine et le résidu d'acide aminé hydrophobe P4 est l'isoleucine.

6. Composé selon la revendication 5, dans lequel les résidus d'acides aminés non acides P1' et P2' sont l'isoleucine et la valine, respectivement.

7. Composé selon la revendication 1, qui contient la séquence de sites de clivage Ile-Lys-Pro-Arg-P1'-P2', dans laquelle chacun des sites Pl' et P2' est indépendamment un acide aminé non acide.

8. Composé selon la revendication 7, qui contient la séquence de sites de clivage Thr-Thr-Lys-Ile-Lys-Pro-Arg-Ile-Val.

9. Composé selon la revendication 8, dans lequel la séquence Pro(559)-Gly(560) du plasminogène de type sauvage est remplacée par la séquence Thr-Thr-Lys-Ile-Lys-Pro et Val(562) est remplacé par Ile.

10. Composé selon la revendication 7, qui contient la séquence de sites de clivage Ala-Thr-Thr-Lys-Ile-Lys-Pro-Arg-Ile-Val.

11. Composé selon l'une quelconque des revendications 1 à 4, dans lequel le résidu d'acide aminé alcalin P3 est un résidu d'arginine et le résidu d'acide aminé hydrophobe P4 est la leucine.

12. Composé selon la revendication 11, dans lequel les résidus d'acides aminés non acides P1' et P2' sont tous deux de la valine.

13. Composé selon la revendication 1, qui contient la séquence de sites de clivage Leu-Arg-Pro-Arg-P1'-P2', dans laquelle chacun des sites P1' et P2' est indépendamment un acide aminé non acide.

14. Composé selon la revendication 13, qui contient la séquence de sites de clivage Val-Glu-Leu-Gln-Gly-Leu-Arg-Pro-Arg-Val-Val.

15. Composé selon la revendication 14, dans lequel la séquence Pro(559)-Gly(560) du plasminogène de type sauvage est remplacée par la séquence Val-Glu-Leu-Gln-Gly-Leu-Arg-Pro.

16. Composé selon la revendication 13, qui contient la séquence de sites de clivage Gly-Gln-Lys-Thr-Leu-Arg-Pro-Arg-Val-Val.

17. Composé selon la revendication 16, dans lequel la séquence Pro(559)-Gly(560) du plasminogène de type sauvage est remplacée par la séquence Gly-Gln-Lys-Thr-Leu-Arg-Pro.

18. Composé selon la revendication 13, qui contient la séquence de sites de clivage Ala-Gly-Gln-Lys-Thr-Leu-Arg-Pro-Arg-Val-Val.

19. Composé selon la revendication 13, qui contient la séquence de sites de clivage Ala-Leu-Arg-Pro-Arg-Val-Val.

20. Composé selon l'une quelconque des revendications 1 à 19, qui comprend, en plus de ladite séquence de sites de clivage, des additions, des délétions ou des substitutions par rapport au plasminogène de type sauvage.

21. Composé selon la revendication 20, dans lequel un ou les deux acides aminés correspondant à Cys(558) et à Cys(566) du plasminogène de type sauvage est (sont) remplacé(s) ou amputé(s).

22. Composé selon la revendication 21, dans lequel un ou les deuxdits résidus est (sont) remplacé(s) par un ou des résidus d'alanine ou de sérine.

23. Composé selon l'une quelconque des revendications 20 à 22, dans lequel lesdites additions, délétions ou substitutions de la séquence se situent dans le domaine du composé correspondant au domaine de la sérine protéase du plasminogène de type sauvage (SEQ ID n° 2).

24. Composé selon l'une quelconque des revendications 20 à 22, dans lequel lesdites additions. délétions ou substitutions de la séquence se situent en dehors du domaine du composé correspondant au domaine de la sérine protéase du plasminogène de type sauvage (SEQ ID n° 2).

25. Composé selon la revendication 23, dans lequel lesdites additions, délétions ou substitutions de la séquence se trouvent dans un ou plusieurs résidus à proximité spatiale étroite, autre que la proximité de séquence, d'un site d'interaction entre le composé et l'antiplasmine.

26. Composé selon la revendication 25, qui a une ou plusieurs des mutations suivantes : Glu-62 en Lys ou Ala, Ser-17 en Leu, Arg 19 en Glu ou Ala, ou Glu-45 en Lys, Arg ou Ala, du domaine de la sérine protéase du plasminogène de type sauvage (SEQ ID n° 2).

27. Composé selon la revendication 23, dans lequel lesdites additions, délétions ou substitutions de la séquence se trouvent dans un ou des résidus d'une région correspondant aux résidus 22 ou 24 du domaine de la protéase de la plasmine (en utilisant la numérotation de la SEQ ID n° 2).

28. Composé selon la revendication 27, qui a une ou les deux mutations suivantes : Phe(583) en Arg ou Mét(585) en Arg.

29. Composé selon la revendication 25, dans lequel le résidu d'acide aminé correspondant à Glu(606) du plasminogène de type sauvage est substitué par un résidu de lysine.

30. Composé selon la revendication 25, dans lequel le résidu d'acide aminé correspondant à Glu(623) du plasminogène de type sauvage est substitué par un résidu de lysine.

31. Composé selon la revendication 24 correspondant à un lys-plasminogène dans lequel le domaine N-terminal a été amputé.

32. Composé selon la revendication 24, dans lequel les résidus d'acides aminés correspondant aux résidus d'acides aminés 68, 77 ou 78 de l'extrémité amino du plasminogène de type sauvage ont été amputés.

33. Composé selon la revendication 24, dans lequel un ou plusieurs domaines kringle correspondant aux domaines kringle du plasminogène de type sauvage sont amputés.

34. Composé selon la revendication 24, contenant une ou les deux mutations suivantes : Asp(137) en Ser ou Asp(139) en Ser.

35. Composé selon la revendication 1, dans lequel la séquence Pro(559)-Gly(560) du plasminogène de type sauvage est remplacée par la séquence Thr-Thr-Lys-Ile-Lys-Pro; Val(562) est remplacé par Ile; Glu(606) est remplacé par Lys et Glu(623) est remplacé par Lys.

36. Composé selon la revendication 1, dans lequel la séquence Cys(558)-Pro(559)-Gly(560) du plasminogène de type sauvage est remplacée par la séquence Ala-Gly-Gln-Lys-Thr-Leu-Arg-Pro et Cys(566) est remplacé par Ala.

37. Composé selon la revendication 1, dans lequel la séquence Cys(558)-Pro(559)-Gly(560) du plasminogène de type sauvage est remplacée par la séquence Ala-Leu-Arg-Pro et Cys(566) est remplacé par Ala.

38. Composé selon la revendication 1, dans lequel la séquence Cys(558)-Pro(559)-Gly(560) du plasminogène de type sauvage est remplacée par la séquence Ala-Thr-Thr-Lys-lle-Lys-Pro; Val(562) est remplacé par Ile; et Cys(566) est remplacé par Ala.

39. Acide nucléique codant pour un composé selon l'une quelconque des revendications 1 à 38.

40. Acide nucléique selon la revendication 39, qui est un vecteur.

41. Vecteur selon la revendication 40, qui est un plasmide, un cosmide ou un phage.

42. Acide nucléique selon la revendication 40 ou 41, comprenant une première séquence d'acides nucléiques codant pour une protéine ou mettant en oeuvre un site de clonage, reliée de manière opérationnelle à une deuxième séquence d'acides nucléiques contenant une séquence promotrice et activatrice forte de promoteurs et de renforçateurs dérivée du cytomégalovirus humain, une troisième séquence d'acides nucléiques codant pour une séquence de polyadénylation dérivée du SV40 et une quatrième séquence d'acides nucléiques codant pour un marqueur sélectionnable exprimé à partir d'un promoteur de SV40 et ayant un signal supplémentaire de polyadénylation du SV40 à l'extrémité 3' de la séquence de marqueur sélectionnable.

43. Cellule ou lignée cellulaire transformée par l'acide nucléique selon l'une quelconque des revendications 39 à 42.

44. Cellule selon la revendication 43. qui est une cellule d'ovaire de hamster de Chine (CHO) mammifère.

45. Cellule ou ligne de cellules selon la revendication 43, qui est sélectionnée dans le groupe comprenant une lignée cellulaire NSO de myélome de souris, une cellule COS ou une cellule BHK.

46. Composé selon l'une quelconque des revendications 1 à 35 susceptible d'être utilisé dans la prophylaxie et/ou le traitement d'états dus à un déséquilibre entre la coagulation et la fibrinolyse.

47. Utilisation d'un composé selon l'une quelconque des revendications 1 à 38 pour la préparation d'un agent destiné à la prophylaxie et/ou au traitement d'états dus à un déséquilibre entre la coagulation et la fibrinolyse.

48. Composition pharmaceutique ou vétérinaire comprenant un ou plusieurs analogues de plasminogène modifiés selon l'une quelconque des revendications 1 à 38 conjointement avec un véhicule acceptable au plan pharmaceutique ou vétérinaire.
